# EUROPEAN PATENT SPECIFICATION

(11) **EP 1 824 383 B1**
(45) Date of publication and mention of the grant of the patent: **05.12.2018**
(21) Application number: 05853829.9
(22) Date of filing: 13.12.2005
(51) Int. Cl.: A61B 5/04, A61B 5/00

(54) **METHOD AND APPARATUS FOR TRANSFER OF CAPTURED ELECTROCARDIOGRAM DATA**
VERFAHREN UND VORRICHTUNG ZUR ÜBERTRAGUNG ERFASSTER ELEKTROKARDIOGRAMMDATEN
PROCEDE ET DISPOSITIF POUR TRANSFERER DES DONNEES D'ELECTROCARDIOGRAMME CAPTUREES

(30) Priority: 13.12.2004 US 634994 P
(43) Date of publication of application: 29.08.2007
(73) Proprietor: Cardiocore Lab, Inc., Bethesda, MD 20817 (US)
(72) Inventor: SATIN, Scott, L., Washington, DC 20015 (US); COCHRAN, Robert, G., New Market, Maryland 21774 (US); PATEL, Nirmal, R., Croydon, Pennsylvania 19021 (US)
(74) Representative: Graf von Stosch, Andreas
(86) International application number: PCT/US2005/045003
(87) International publication number: WO 2006/065784

(56) References cited:
- US-A- 4 531 527
- US-A- 5 936 539
- US-B1- 6 366 871
- US-B1- 6 416 471
- US-B1- 6 579 231

## Description

### BACKGROUND OF ILLUSTRATIVE EMBODIMENTS OF THE INVENTION

The heart is a pump comprised of muscle tissue that responds to electrical stimulation. A heartbeat is a precisely controlled event that relies on synchronization between the atrial and ventricular chambers to maximize pumping efficiency. The sinoatrial node, which is located in the right atrium of the heart, generates the electrical stimulus. In a healthy person, the sinoatrial node normally generates electrical stimulus signals at a 60-100 Hz rate, and the waves of myocardial excitation and contraction spread throughout the heart in well-defined manner. The electrical stimulus signals cause contractions in the heart's chambers, thereby pumping blood through the chambers. The left and right atria of the heart contract first and for a brief time, and then the left and right ventricles contract for a brief time. Normal heart rhythm is referred to as "sinus" rhythm, because it originates in the sinoatrial node (also referred to as the sinus node). The electrical stimulus signal output by the sinoatrial node is first sent to the left and right atria, then through the atrioventricular node and into the left and right ventricles.

An electrocardiogram (ECG) measures the heart's electrical activity. Electrodes are placed at specific locations on the body to capture a tracing of the heart's electrical activity. The electrical activity resulting from heart depolarization and heart repolarization is recorded by each lead. The ECG is a summation of the information recorded from each lead. The captured ECG reflects the direction of electrical current flow, and the magnitude of the muscle that is depolarized. Therefore, when the atria depolarize (and contract) the ECG tracing is smaller as compared to when the ventricles contract, since the atria are much smaller than the ventricles. Ventricle repolarization is in the same direction (positive) as ventricle depolarization. Although an ECG is positive during membrane depolarization and negative during repolarization, the direction with respect to ventricles is the same since ventricles depolarize from the inside to the outside (endocardium to epicardium), while repolarization occurs in the opposite direction.

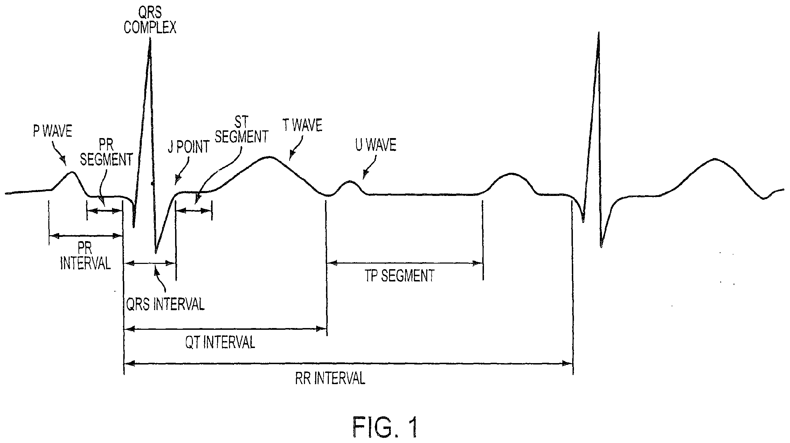

Referring to FIG. 1, an ECG tracing is illustrated. The cardiac cycle begins with a P-wave, wherein the spontaneously firing cells in the sinoatrial node reach a threshold and generate action potentials. A wave of depolarization spreads to the left and downward though left and right atria, which is labeled in FIG. 1 as the "P wave." The atria that were hyperpolarized suddenly become depolarized, and the ECG records a positive deflection. When the left and right atria become depolarized, the ECG returns to zero. The electrical current passes through the atrioventricular node, causing a delay of about one-tenth of a second. Due to the small mass of the atrioventricular node, the ECG tracing does not record any electrical activity. When the atrioventricular node is depolarized, it triggers depolarization of the Purkinje fibers. The Purkinje fibers spread the electrical current throughout the left and right ventricles, thereby causing depolarization across each ventricle simultaneously. Since the tissue mass of the Purkinje fibers is small, the ECG tracing does not record any electrical activity. The passing of the electrical current through the atrioventricular node and the Purkinje fibers is labeled in FIG. 1 as the "PR segment."

The depolarization of the left and right ventricles is referred to as the "QRS complex," and FIG. 1 is labeled as such. The QRS complex is quite large since the left and right ventricle tissue is large in comparison to the sinoatrial node. The three peaks are indicative of the manner in which the electrical current spreads through the left and right ventricles, *i.e*., from inside to outside, and because the tissue mass of the left ventricle is greater than the tissue mass of the right ventricle. The complete depolarization of the left and right ventricles indicates that the QRS complex has terminated.

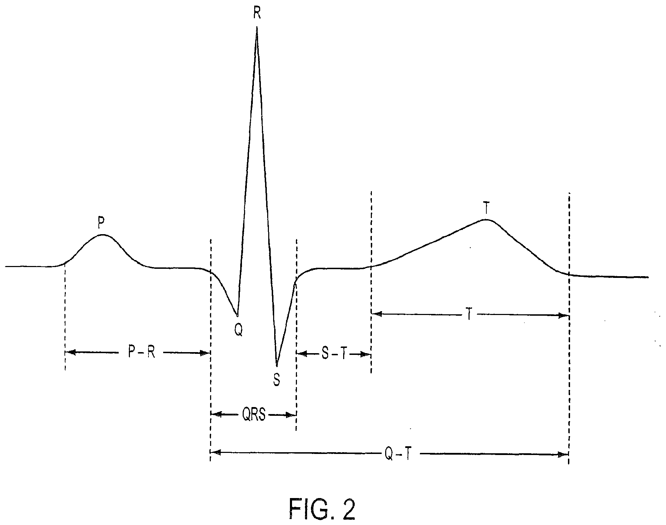

Referring to FIG. 2, the points of the QRS complex are labeled. As noted above, the QRS complex is indicative of the depolarization of the left and right ventricles. The ventricular depolarization begins at a left side of the intraventricular septum, and the peak of this depolarization is shown by the "Q" peak of the QRS complex. The ventricular depolarization spreads from the endocardial surface of the left ventricle to the epicardial surface of the left ventricle, and is shown by the "R" peak of the QRS complex. The spread of the ventricular depolarization to the right ventricle is shown by the "S" peak of the QRS complex.

The segment labeled "T wave" indicates repolarization of the left and right ventricles. Although the left and right ventricles are repolarizing, the T wave is positive, since the heart repolarizes from outside to inside, which is the opposite direction of depolarization (inside to outside). The completion of the T wave signals marks the end of the cardiac cycle.

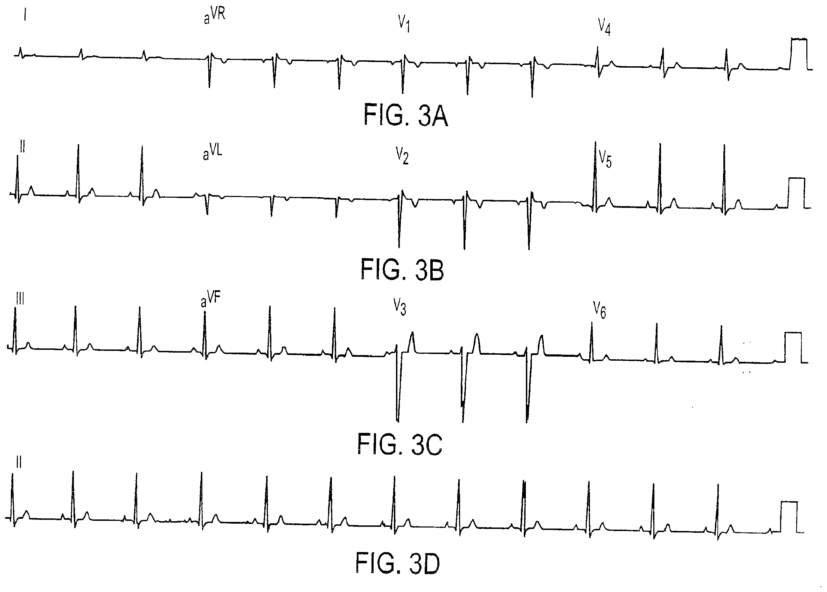

Referring to FIG. 3, the captured tracing of electrical activity is printed out on a paper tape or is presented on a display. Anomalies in an ECG are indicative of various heart-related conditions, such as ischemia, myocardial infarction, conduction disorder, electrolyte disturbance, pericarditis, valve disease or enlarged heart. Certain arrhythmias might occur only on an intermittent basis, or only if certain psychological or physical factors (*i.e*., stress, fatigue, etc.) are present. Since a typical ECG tracing is only a few minutes in length, arrhythmias of this type are difficult to capture. A more lengthy ECG tracing, referred to as a Holter monitor, is used to capture any arrhythmias or other abnormal activity. The Holter monitor may record a heart's activity over a period of several days.

Referring to FIG. 1, one of the segments that is measured is the referred to as the QT interval, and the QT interval indicates the duration of the electrical activity that controls contraction of the cells of the heart muscle. The QT interval represents the duration of ventricular depolarization and subsequent repolarization, beginning at the initiation of the Q wave of the QRS complex and ending where the T wave returns to the isoelectric baseline. QT interval prolongation creates an electrophysiological environment that favors the development of cardiac arrhythmias, most commonly torsade de pointes, but possibly other ventricular arrhythmias as well. Long QT syndrome identifies a condition wherein there exists an abnormally long QT interval on the ECG tracing. The term "congenital long QT" refers to a long QT interval that is inherited. The inherited form occurs due to irregularities in particular heart cell proteins, and, of course, these protein irregularities are caused by abnormalities in the genes that produce those proteins. The term "acquired long QT" refers to a long QT interval that is brought about by drugs or anomalous levels of the salts within blood, *e*.*g*., potassium and magnesium.

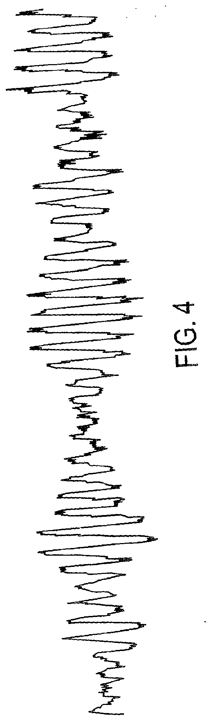

Although a person might have an unremarkable QT interval under normal conditions, that person might develop a prolonged QT or suffer torsades de pointes (TdP) when taking certain medications. As shown in FIG. 4, TdP refers to the characteristic appearance of the electrocardiogram indicative of a rhythm abnormality, and typically occurs in the setting of a prolonged QT interval on the electrocardiogram. TdP is a polymorphic ventricular tachyarrhythmia that manifests on the ECG tracing as continuous twisting of the vector of the QRS complex around the isoelectric baseline. A feature of TdP is pronounced prolongation of the QT interval in the sinus beats preceding the arrhythmia. TdP can degenerate into life-threatening cardiac rhythms that can result in blackouts or sudden death. Measurement of the QT interval on the ECG tracing is still the main method of determining whether a person has long QT interval syndrome, whether inherited or acquired.

Non-antiarrhythmic drugs can have an undesirable side effect of causing delayed cardiac repolarization. Due to its relationship to heart rate, the QT interval is normalized into a heart rate independent "corrected" value known as the QT_{c} interval, which represents the QT interval at a standardized heart rate (essentially the QT interval at a heart rate of 60 bpm). Several drugs that have caused TdP clearly increase both the absolute QT interval and the QT_{c} interval.
US 6,416,471 B1 discloses a portable remote patient telemonitoring system having a cordless, disposable sensor band with sensors and transmission circuitry. A small signal transfer unit that can either be worn by the patient or positioned nearby receives data from the sensor band, which it then forwards to a base station. US 4,531,527 discloses a cardiac monitoring system including a plurality of patient-worn units and one or more office units. A patient unit detects and analyzes a patient's EKG. The analyzed data is sent over a standard voice-grade telephone line or other suitable communication channel to an office which prepares a patient report for a physician. US 6,366,871 B1 discloses ambulatory patient monitoring apparatus including a portable housing including at least one physiological data input device and cellular telephone communications circuitry for communicating the physiological data to a central health monitoring station.

### SUMMARY OF ILLUSTRATIVE EMBODIMENTS OF THE INVENTION

Illustrative, non-limiting embodiments of the present invention overcome various disadvantages. In addition, the present invention is not required to overcome these disadvantages, and an illustrative, non-limiting embodiment of the present invention may not overcome any problems.

According to one aspect, a method for transfer of electrocardiograph data between a remote processor connected to a central processor via a network wherein identification information concerning a test subject associated with the electrocardiograph data is entered comprises recording demographic data associated with the test subject on a portable storage medium (63) containing the electrocardiograph data; coupling a portable storage medium containing the electrocardiograph data to the remote processor; extracting the electrocardiograph data from the portable storage medium and transmitting the identification information and the electrocardiograph data from the remote processor to the central processor; and transmitting a release message from the central processor to the remote processor to enable erasure of the portable storage medium, wherein the transmission of the release message is subsequent to the successful storage of the electrocardiograph data on the central processor.

According to another aspect, a computer program product for transfer of electrocardiograph data between a remote processor connected to a central processor via a network comprises a computer readable storage medium; and computer executable code embodied on the computer readable medium, wherein the computer executable code, when executed, performs the steps of: providing for entry of identification information concerning a test subject associated with electrocardiograph data resident on a portable storage medium coupled to the remote processor; recording demographic data associated with the test subject on the portable storage medium containing the electrocardiograph data; extracting the electrocardiograph data from the portable storage medium and transmitting the identification information and the electrocardiograph data from the remote processor to the central processor; and transmitting a release message from the central processor to the remote processor to enable erasure of the portable storage medium, wherein the transmission of the release message is subsequent to the successful storage of the electrocardiograph data on the central processor.

According to yet another aspect, an apparatus for transferring electrocardiograph data from a portable storage medium comprises: a portable storage medium for storing electrocardiograph data and demographic data associated with a test subject; a remote processor for extracting the electrocardiograph data from the portable storage medium, and for transmitting identification information concerning the test subject and the extracted electrocardiograph data; a central processor for storing the extracted electrocardiograph data and transmitting a release message to the remote processor to enable erasure of the portable storage medium; and a network connecting the remote processor and the central processor, wherein the central processor is configured to receive the identification information and electrocardiograph data transmitted by the remote processor via the network, and the remote processor is configured to receive the release message from the central processor via the network.

Additional aspects and advantages of illustrative embodiments of the invention will be set forth in part in the description that follows or may be learned by practice of the embodiments. The aspects and advantages of the embodiments may be realized and attained by means of the instrumentalities and combinations particularly pointed out in the appended claims. The embodiments serve only for illustrative purposes, the invention being defined by the appended claims.

### BRIEF DESCRIPTION OF THE DRAWINGS

The above and other features and advantages of illustrative, non-limiting embodiments of the present invention will become more apparent from the following description. The accompanying drawings, which are incorporated in and constitute a part of this specification, illustrate exemplary embodiments of the invention and, together with the description, serve to explain the aspects, advantages and principles of the embodiments. In the drawings:
FIG. 1 is an illustration of an ECG tracing that identifies the various segments of an electrical profile of a normal heartbeat;
FIG. 2 is an illustration of an ECG tracing that identifies the various peaks of an electrical profile of a normal heartbeat;
FIG. 3 is an illustration of the output from a 12-lead Holter monitoring device;
FIG. 4 is an illustration of an ECG tracing showing torsades de pointes;
FIG. 5 is an illustration of an exemplary, non-limiting computer system for use as a remote processor system;
FIG. 6 is an illustration of an exemplary, non-limiting networked computer system for use with embodiments of the present invention;
FIG. 7 is an exemplary flowchart illustrating the process flow for the transfer of electrocardiograph data from a remote processor to a central processor;
FIG. 8 is an exemplary flowchart illustrating a process flow for the entering of identification information for new and existing test subjects for a drug test protocol;
FIG. 9 is an exemplary illustration of a screen containing a release list that a remote processor displays;
FIG. 10 is an exemplary illustration of a screen that a remote processor displays to prompt an operator to enter a test subject's initials;
FIG. 11 is an exemplary illustration of a screen that a remote processor displays to prompt an operator to enter a test subject's date of birth; and
FIG. 12 is an exemplary illustration of a screen that a remote processor displays to prompt an operator to enter an identification number of a portable storage medium;
FIG. 13 is an exemplary illustration of a screen that a remote processor displays to prompt an operator to select a type of visit;
FIG. 14 is an exemplary illustration of a screen that a remote processor displays to prompt an operator to verify an existing test subject's demographic information prior to sending the information to a central processor;
FIG. 15 is an exemplary illustration of a screen that a remote processor displays to prompt an operator to select an existing test subject;
FIG. 16 is an exemplary illustration of a screen that a remote processor displays to prompt an operator to verify a selected test subject;
FIG. 17 is an exemplary illustration of a screen that a remote processor displays to prompt an operator to select a status of a type of transmission of data from a remote processor to a central computer;
FIG. 18 is an exemplary illustration of a screen that a remote processor displays to inform an operator of a status of a current transmission to a central computer;
FIG. 19 is an exemplary illustration of a screen that a remote processor displays to inform an operator of a status of pending transmissions to a central computer;
FIG. 20 is an exemplary illustration of a screen that a remote processor displays to inform an operator of a status of completed transmissions to a central computer;
FIG. 21 is an exemplary illustration of a screen that a remote processor displays to prompt the operator to select a drug test protocol;
FIG. 22 is an exemplary illustration of a screen that a remote processor displays to prompt the operator to select whether or not the test subject is a new test subject or an existing test subject;
FIG. 23 is an exemplary illustration of a screen that a remote processor displays to prompt the operator to enter the test subject's identification number;
FIG. 24 is an exemplary illustration of a screen that a remote processor displays to prompt the operator to verify that a facsimile has been sent and that a portable storage medium is connected to the remote processor; and
FIG. 25 shows a non-limiting example of a system overview in accordance with an embodiment of the invention.

### DETAILED DESCRIPTION OF THE ILLUSTRATIVE, NON-LIMITING EMBODIMENTS OF THE INVENTION

Illustrative, non-limiting embodiments of the present invention will now be described more fully with reference to the accompanying drawings. The same elements are given the same reference numerals.

A general example of a computer that can be used in accordance with the described embodiment will be described below.

The computer comprises one or more processors or processing units, a system memory and a bus that couples various system components comprising the system memory to processors. The bus can be one or more of any of several types of bus structures, comprising a memory bus or memory controller, a peripheral bus, an accelerated graphics port and a processor or local bus using any of a variety of bus architectures. The system memory comprises read only memory (ROM) and random access memory (RAM). A basic input/output system (BIOS) containing the routines that help to transfer information between elements within the computer, such as during boot up, is stored in the ROM or in a separate memory.

The computer further comprises a hard drive for reading from and writing to one or more hard disks. Some computers can comprise a magnetic disk drive for reading from and writing to a removable magnetic disk and an optical disk drive for reading from or writing to a removable optical disk, such as a CD ROM or other optical media. The hard drive, the magnetic disk drive and the optical disk drive are connected to the bus by an appropriate interface. The drives and their associated computer-readable media provide nonvolatile storage of computer-readable instructions, data structures, program modules and other data for the computer. Although the exemplary environment described herein employs a hard disk, a removable magnetic disk and a removable optical disk, it should be appreciated by those skilled in the art that other types of computer-readable media which can store data that is accessible by a computer, such as magnetic cassettes, flash memory cards, digital video disks, random access memories (RAMs), read only memories (ROMs), carrier waves, etc. may also be used in the exemplary operating environment.

A number of program modules may be stored on the hard disk, magnetic disk, optical disk, ROM or RAM, comprising an operating system, at least one or more application programs, other program modules and program data. In some computers, an operator might enter commands and information into the computer through input devices such as a keyboard and a pointing device. Other input devices may comprise a microphone, a joystick, a game pad, a satellite dish and/or a scanner. In some instances, however, a computer might not have these types of input devices. These and other input devices are connected to the processing unit through an interface coupled to the bus. In some computers, a monitor or other type of display device might also connect to the bus via an interface, such as a video adapter. Some computers, however, do not have these types of display devices. In addition to the monitor, the computers might comprise other peripheral output devices such as speakers and printers.

The computer can, but need not, operate in a networked environment using logical connections to one or more remote computers. The remote computer may be another personal computer, a server, a router, a network PC, a peer device or other common network node, and typically comprises many or all of the elements described above relative to the computer. The logical connections to the computer may comprise a local area network (LAN) and a wide area network (WAN). Such networking environments are commonplace in offices, enterprise-wide computer networks, intranets, and the Internet.

When used in a LAN networking environment, the computer is connected to the local network through a network interface or adapter. When used in a WAN networking environment, the computer typically comprises a modem or other means for establishing communications over the wide area network, such as the Internet. The modem, which may be internal or external, is connected to the bus via a serial port interface. In a networked environment, program modules depicted relative to the computer, or portions thereof, may be stored in the remote memory storage device. It will be appreciated that the network connections shown are exemplary and other means of establishing a communications link between the computers may be used.

Generally, the data processors of the computer are programmed by means of instructions stored at different times in the various computer-readable storage media of the computer. Programs and operating systems are typically distributed, for example, on floppy disks or CD-ROMs. From there, they are installed or loaded into the secondary memory of the computer. At execution, they are loaded at least partially into the computer's primary electronic memory. Non-limiting embodiments of the invention described herein comprises these and other various types of computer-readable storage media when such media contain instructions or programs for implementing the steps described below in conjunction with a microprocessor or other data processor. Embodiments also comprise the computer itself when programmed according to the methods and techniques described below.

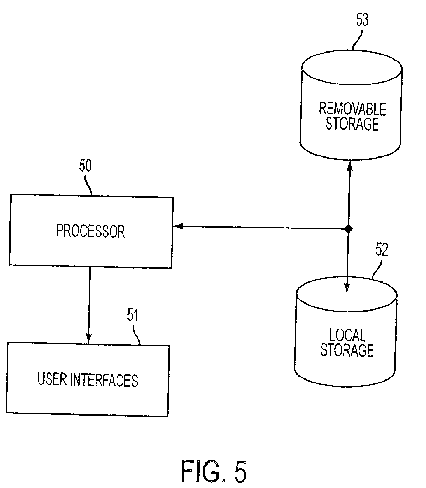

Referring to FIG. 5, a computer for use in a non-limiting embodiment of the present invention is illustrated. The computer comprises a processor 50, user interfaces 51 and local storage 52. As described above, the processor 50 may comprise one or more processors, and the user interfaces 51 may comprise monitors, keyboards, mice, touch-screens, etc. The processor 50 is connected to the local storage 52 via a bus (or busses) as described above, and the local storage 52 itself may comprise various types of disk memory, electronic memory (*i.e*., RAM, ROM) or various combinations thereof. The processor 50 may also access the removable storage 53, which itself may comprise various types of data storage machines and/or server machines. A Holter recording file, also referred to as electrocardiograph data, is stored in either the remote storage 53 or the local storage 52, and the processor 50 accesses the Holter recording file therefrom.

As is known, electrocardiograph data (*i.e*., a Holter recording file) is stored in a variety of different file formats, such as FDA XML, Mortara XML as exported from E-Scribe, and GE® MUSE®. Typically, each Holter recording file will contain 24 or 48 hours of 12-lead data at 1k samples per second. It is foreseen that an embodiment of the present invention will be able to handle a Holter recording file of at least 48 hours × 12 leads × 1k samples per second, but it is also foreseen that an embodiment of the present invention will not have any intrinsic limitations that prevent the handling longer recordings or recordings taken at higher and/or lower sampling rates.

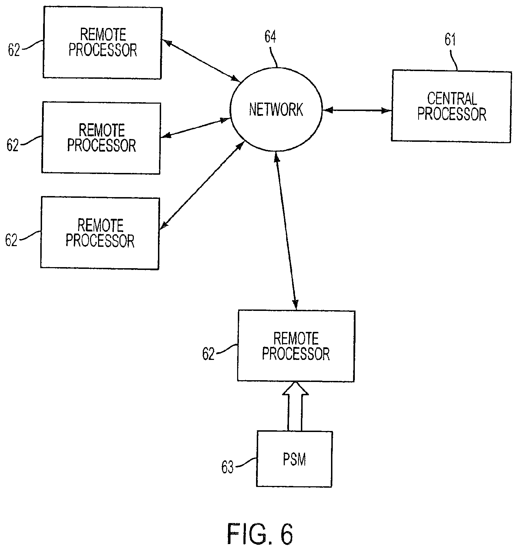

Referring to FIG. 6, an exemplary embodiment of the present invention is shown. A central processor 61 is connected to a network 64, and a remote processor 62 is coupled to the network 64 as well. In practice, a plurality of remote processors 62 would be coupled to the network 64 in various drug testing sites spread geographically across one country or several countries.

A portable storage medium 63 stores electrocardiograph data. The portable storage medium 63 may be a device comprising a memory circuit (*e.g*., flash memory) and interfacing circuitry, such as, but not limited to, a Universal Serial Bus interface. One of skill in the art will appreciate that other media and interfacing techniques can be used as well. The portable storage medium 63 may also be a device that uses a magnetic or optical storage medium, with or without accompanying interfacing circuitry. The electrocardiograph data is transferred from the Holter device to the portable storage medium 63 using data transfer techniques known to one skilled in the art.

As shown in FIG. 6, the portable storage medium 63 is coupled to the remote processor 62 via cabling or inserting the portable storage medium 63 into a connector that is compatible with the interfacing technique used to transfer data from the portable storage medium 63. For example, if the portable storage medium 63 used FIREWIRE for data transfer purposes, the connector on the remote processor 62 is adapted to handle data transmissions using FIREWIRE.

Prior to any transfer of electrocardiograph data from the remote processor 62 to the central processor 61, information relating to the test subject, whose electrocardiograph data is to be stored on a particular portable storage medium 63, is stored in a database resident on the central processor 61 and/or the remote processor 62. The information stored in the database may comprise at least an identification number associated with the test subject, the initials of the test subject, date of birth of the test subject, an identification number of the portable storage medium 63 associated with the test subject and gender of the test subject.

The central processor 61 may perform various administrative functions. For example, the processor 61 may create lists of registered drug test sites having a remote processor 62 and may allow an operator to maintain the lists of registered sites. Also, the processor 61 may enable an operator to create and enter drug test protocol identifiers for each drug test site and maintain these protocols.

Additionally, the central processor 61 may allow an operator to create a database of authorized operators of the remote processors 62 at each drug test site and to maintain the database. For example, the database may include various data, including but not limited to the username, password, email, full name, contact telephone number, etc. for each authorized operator. Also, the processor 61 may store the number of test subjects that have visited and/or submitted data at each drug test site.

Furthermore, in one embodiment, the portable storage medium 63 is relatively expensive. Thus, instead of utilizing a new storage medium 63 for each test subject, an operator at a drug testing site may store data on the medium 63 for a first test subject, transfer the data to the central processor 61, and erase the data from the medium 63 after the transfer. Then, the operator at the drug testing site may use the same portable storage medium 63 to store data for a second test subject and transfer the data to the processor 61.

However, in the embodiment, a substantial amount of time is required to obtain the data for each of the test subjects and to transfer the data to the central processor 61. Therefore, if an error occurs during the transfer of data from the remote processor 62 to the central processor 61 or during the storage of data on the processor 61, and if an operator subsequently erases the data from the medium 63, the data may be lost. Accordingly, the central processor 61 may implement a process to prevent such a data loss. For example, as noted above, the remote processor 62 transfers the identification number of the portable storage medium 63 along with the data for the test subject. After the central processor 61 successfully retrieves the data from the processor 62 and stores it, it may create a "release list" of all of the media 63 from which data has been successfully retrieved and stored. Then, the central processor 61 may transmit all or part of this release list to the remote processors 62 from which it successfully received and stored data.

In one embodiment, the remote processor 62 receives at least a portion of the release list that comprises the identification numbers of the media 63 that contained data that the remote processor 62 transferred to the central processor 61. In such case, each entry in the list corresponds to a release command that enables an operator of the processor 62 to erase the data from the appropriate storage medium 63 having the listed identification number so that the operator can reuse the storage medium 63. Alternatively, the central processor 61 may not transmit the release list to the processor 62 and instead, may transmit a release command, which includes the identification number of the medium 63 that may be erased and which authorizes the operator of the processor 62 to erase the data from the medium 63. In a further implementation, the processor 62 prevents an operator from erasing the data on the storage medium 63 prior to receiving the release list or the release command.

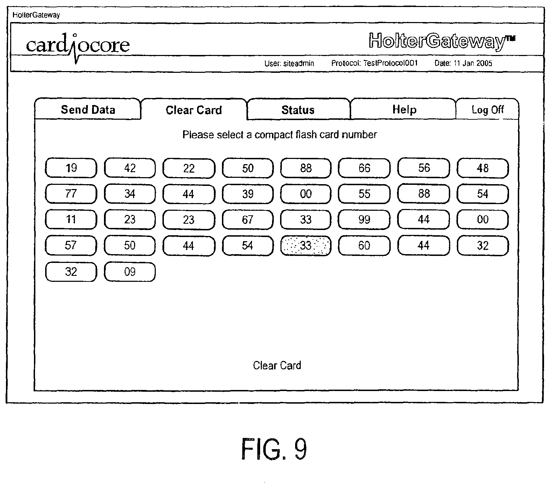

FIG. 9 shows an illustrative example of a release list that the remote processor 62 displays. As shown in the figure, the list contains the identification numbers of various storage media (*e*.*g*., compact flash cards) 63 that the central processor 61 has authorized to be erased. Upon displaying the list, an operator of the remote processor 62 can select one of the identification numbers (*e.g*., the number "33") and can select the "Clear Card" option to erase the medium 63 having the selected identification number.

In addition to the identification number that appears on the portable storage medium 63, the "volume label" of the portable storage medium 63 is recorded on the central processor 61. In one embodiment, the central processor 61 uses the volume label to confirm that the identification number on the portable storage medium 63 to be cleared corresponds to the actual portable storage medium 63 from which data has been successfully received from the remote processor 62. If both the volume number and the identification number correspond to the medium 63, the central processor 61 authorizes the data on the medium 63 to be erased. On the other hand, if either the volume number or the identification number does not correspond to the medium 63, the processor 61 does not authorize the data to be erased.

To transmit electrocardiograph data from the portable storage medium 63 to the central processor 61, the medium 63 is coupled to the remote processor 62. Then, the operator of the remote processor 62 selects a drug test protocol that is associated with the electrocardiograph data stored on the portable storage medium 63. The remote processor 62 will display the drug test protocols for which it will accept electrocardiograph data. The remote processor 62 may accept electrocardiograph data for only a single drug protocol, or it may accept electrocardiograph data for several drug test protocols.

After confirming at the remote processor 62 that the necessary information related to the test subject has been provided to the database resident on the central processor, the operator of the remote processor 62 selects whether the test subject, who is associated with the portable storage medium 63 presently coupled to the remote processor 62, is a new test subject or an existing test subject.

If the test subject is a new test subject, then the operator at the remote processor 62 enters demographic data associated with the test subject, such as the test subject's identification number, the initials of the test subject, the date of birth of the test subject and the gender of the test subject. The operator also enters the identification number of the portable storage medium 63 associated with the test subject and the type of visit for the test subject. For example, the types of visit may include, but are not limited to, a screening visit, a baseline visit, a "day *n*" visit, and an unscheduled visit.

In the screening visit, an operator determines the test subject's eligibility to participate in particular testing of a drug based on various inclusion and exclusion criteria specified in the drug test protocol. The baseline visit is typically the first visit of a test subject to the drug test site, during which an operator takes an initial 24 hour continuous Holter recording from the test subject. The operator then extracts the ECG data for the test subject from the Holter recording. The "day *n*" visit is a visit that is scheduled a predetermined amount of time (*e.g., n* days) after the baseline visit. The predetermined amount of time is determined based on the particular drug test protocol. Finally, the unscheduled visit occurs when a test subject visits the drug testing site at a time that does not coincide with the times that the drug test protocol prescribes.

After the operator has confirmed that the demographic data, the identification number of the portable storage medium 63, and the type of visit are correct, this information is transmitted, along with the electrocardiograph data uploaded from the portable storage medium 63 associated with the test subject, from the remote processor 62 to the central processor 61 via the network 64.

If the test subject is an existing test subject, then the operator selects the appropriate test subject from a list of test subjects that are stored locally on and displayed by the remote processor 62. After an existing test subject is selected, the operator may edit the demographic data associated with the test subject. After confirming selection of a particular test subject, the operator then enters the identification number of the portable storage medium 63 associated with the test subject and the type of visit for the test subject. After the operator has confirmed that the demographic data, the identification number of the portable storage medium 63 and the type of visit are correct, this information is transmitted, along with the electrocardiograph data uploaded from the portable storage medium 63 associated with the test subject, from the remote processor 62 to the central processor 61 via the network 64.

Once the operator has commanded the remote processor 62 to transmit the identification information and the electrocardiograph data for a particular test subject to the central processor 61, the operator can remove the portable storage medium 63 associated with the test subject and proceed with uploading data for another test subject.

In one embodiment, the remote processor 62 creates a file containing the identification information and the electrocardiograph data for a particular test subject and then transmits the file to the central processor 61. Also, in order to ensure that all of the files that the central processor 61 receives from the various remote processors 62 have uniform file names, each remote processor 62 automatically creates a filename for the file in accordance with a predetermined format before it transmits the file. For example, the processor 62 may automatically create the file name such that file name identifies the demographic information, the visit type, and the identification number of the portable storage medium 63 corresponding to the data within the file. As such, the central processor 61 can readily recognize valuable information about the file simply by analyzing the file name. The following file name is just one example of a file name that the remote processor 62 creates:

```
       Username_SiteID_Card#_ProtocolID_VisitType_SubjectID_SubjectInitial
       s_DateOfBirth_DateTransmissionBegins_Gender_DiskVolumeSerialNum
       ber.
```

Also, the file name may be decoded and used to generate an HTML form having a header that is automatically populated with the drug test protocol, drug test site identifier, demographic information, and the visit type. This HTML form may represent a document that confirms that the file has been copied to a location on the central processor 61, that the demographic data matches a test subject in the database of the processor 61, and that the image and other data are readable. In a further implementation, completion of this form triggers the central processor 61 to send a release list, message, or command (as described above) to the remote processor 62 and enables the operator of the processor 62 to erase the portable storage medium 63 and use it again.

After transmission of the electrocardiograph data to the central processor 61, the central processor 61 will determine if the transmitted identification information matches a test subject in its database and, if so, stores the electrocardiograph data in a location associated with the test subject. Subsequent to successful storage of the electrocardiograph data, the central processor 61 sends a release message to the remote processor 62 that enables the portable storage medium 63 to be erased and used again, as described above. The operator couples the portable storage medium 63 to be cleared to the remote processor 62, and commands the remote processor 62 to erase any electrocardiograph data stored on the portable storage medium 63 if a release message has been received for the identification number of the portable storage medium 63.

The operator of the remote processor 62 has the ability to monitor current, pending and completed transmissions of identification data and electrocardiograph data to the central processor 61. When commanded, the remote processor 62 displays information related to a current transmission, such as file name, bytes transferred, total bytes and percentage of file transferred. When commanded, the remote processor 62 displays information related to pending transmission, such as the identification number of the test subject, date of birth of the test subject, and the start date of the transmission. When commanded, the remote processor 62 displays information related to completed transmissions, such as the identification number of the test subject, date of birth of the test subject, initials of the test subject, and the transmission completion time. The above-listed parameters are exemplary in nature, and other parameters can be used in place of or in conjunction with the above-listed parameters.

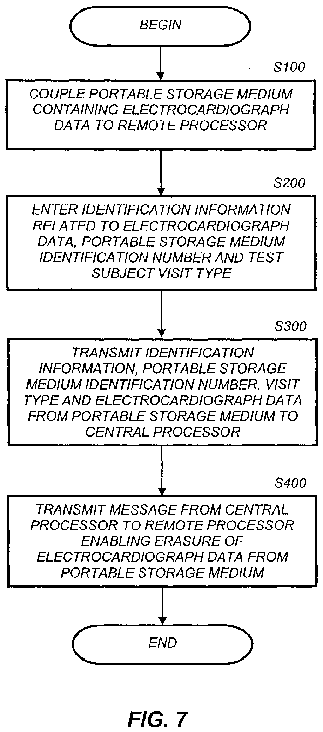

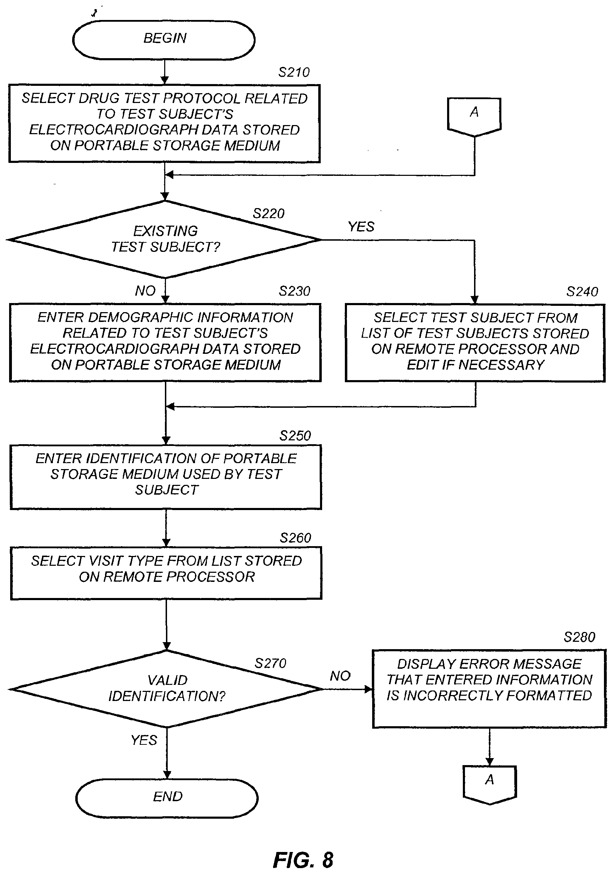

Referring to FIG. 7, a flowchart of the method of a non-limiting embodiment of the present invention is shown. At S100, the portable storage medium 63 is coupled to the remote processor 62. At S200, identification information, the identification number of the portable storage medium 63, and the type of visit associated with the test subject, whose electrocardiograph data is stored on the portable storage medium 63 is entered. The entry of identification information associated with the test subject will be explained in greater detail in FIG. 8 and its accompanying text. At S300, after the identification and other information are entered, all this information is transmitted, along with the electrocardiograph data uploaded from the portable storage medium 63 associated with the test subject, from the remote processor 62 to the central processor 61 via the network 64.

At S400, after the central processor 61 has confirmed that the transmitted identification information matches a test subject in its database and has stored the electrocardiograph data in a location associated with the test subject, the central processor 61 sends a release message to the remote processor 62 that enables the portable storage medium 63 to be erased and used again. After receipt of the release message associated with the identification number of the portable storage medium 63, the remote processor 62 erases any electrocardiograph data stored on the portable storage medium 63.

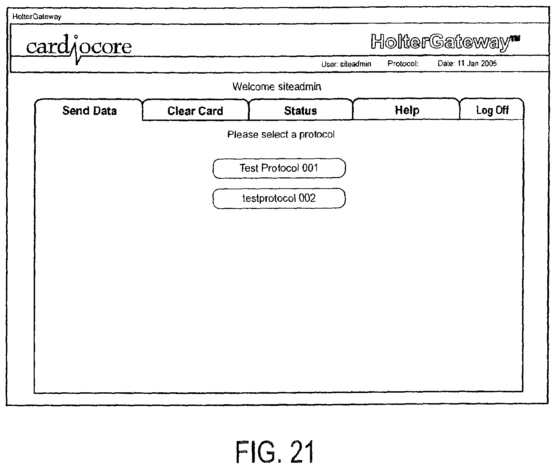

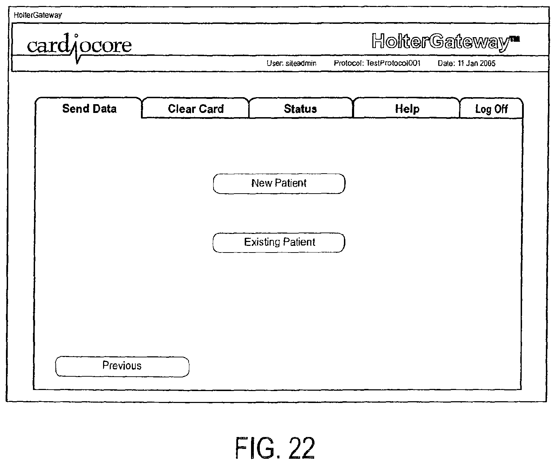

Referring to FIG. 8, the entry of identification information related to a test subject will be illustrated in greater detail. At S210, after the portable storage medium 63 is coupled to the remote processor 62, a drug test protocol that is associated with the electrocardiograph data stored on the portable storage medium 63 is selected. For example, FIG. 21 shows an illustrative example of a screen that the remote processor 62 displays to prompt the operator to select a drug test protocol. Next, at S220, a selection is made whether the test subject (or patient), who is associated with the portable storage medium 63 presently coupled to the remote processor 62, is a new test subject or an existing test subject. FIG. 22 shows an illustrative example of a screen that the remote processor 62 displays to prompt the operator to select whether or not the test subject is a new test subject or an existing test subject.

At S230, if the test subject is a new test subject, demographic data associated with the test subject, such as the test subject's identification number, the initials of the test subject, the date of birth of the test subject, and the gender of the test subject, is entered in a database on the remote processor 62 and/or recorded on the portable recording medium 63. Also, in one embodiment, the processor 62 constrains the manner in which the operator can enter the demographic information so that various operators at one or more drug testing sites enter the data in a uniform manner.

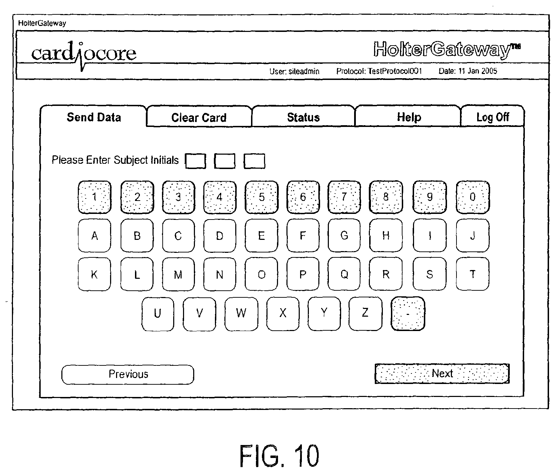

For example, FIG. 10 shows an illustrative example of a screen that the remote processor 62 displays to prompt the operator to enter a test subject's initials. As shown in the figure, the screen forces the operator to enter the initials in a predefined format, such as entering three characters for the initials of the first, middle, and last names, respectively. Alternatively, if a subject does not have a middle name and only has a two initials, the processor 62 may only permit the operator to enter the first initial for the first initial entry, the second initial for the third initial entry, and a predetermined character (*e.g*., a hyphen or an "X") as the middle initial entry.

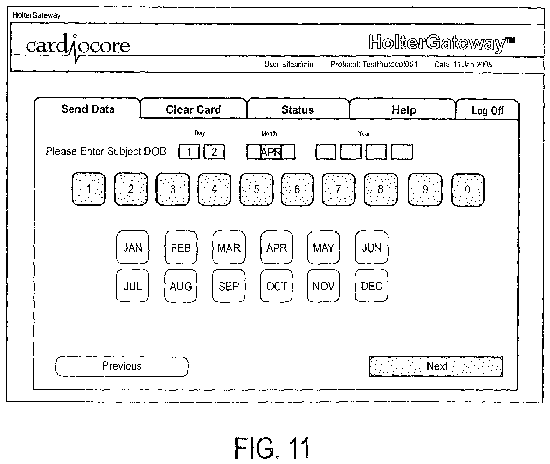

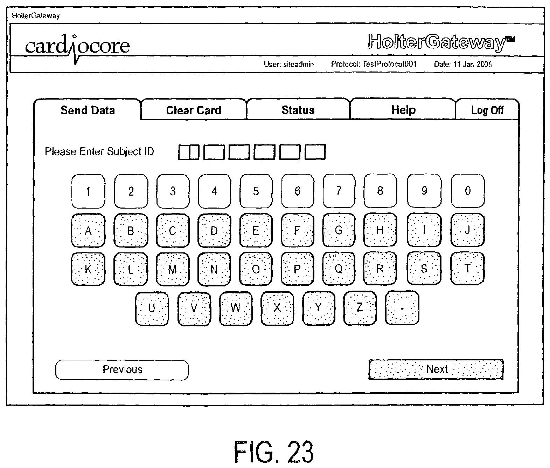

Fig. 11 also shows an illustrative example of a screen that the remote processor 62 displays to prompt the operator to enter a test subject's date of birth. As shown in the figure, the screen forces the operator to enter the date of birth as a two digit day, followed by a three-lettered abbreviation of a month, followed by a four digit year. Also, the remote processor may prevent an operator for entering erroneous data. For example, if the operator enters "31" for the two digit day, the processor may prevent the operator from selecting a month that has less than 31 days. FIG. 23 shows an illustrative example of a screen that the remote processor 62 displays to prompt the operator to enter the test subject's identification number. In any event, after entering the data at S230, control passes to S250.

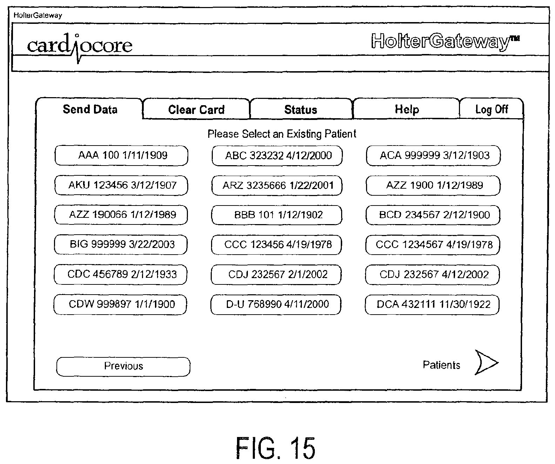

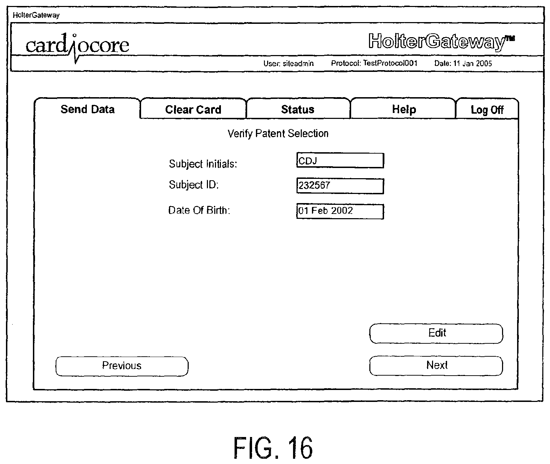

If the test subject is an existing test subject, then at S240, the appropriate test subject is selected from a list of test subjects stored locally on a database of the remote processor 62. FIG. 15 shows an illustrative embodiment of a screen that the processor 62 displays to prompt the operator to select an existing test subject (or patient). Also, as shown in FIG. 16, the remote processor 62 may display a screen requesting the operator to verify the demographic information of the existing test subject. If the information is correct, the operator may select the "Next" option in FIG. 16 to continue. Otherwise, the operator can edit the demographic data associated with the test subject by selecting the "Edit" option in the figure. Control then passes to S250.

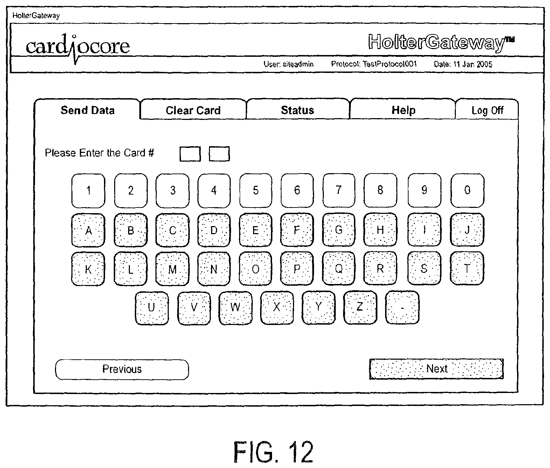

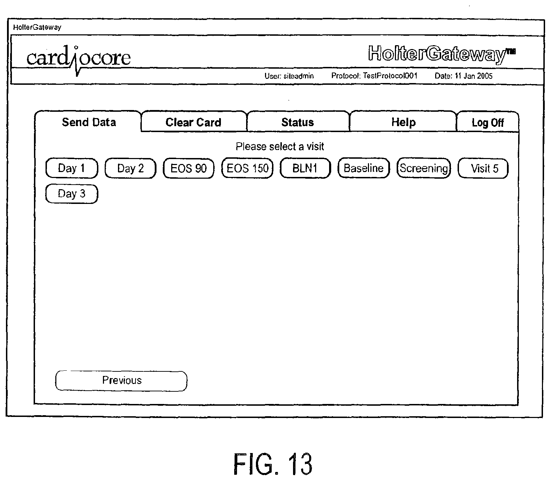

At S250, the identification number of the portable storage medium 63 associated with the test subject is entered into the remote processor 62. FIG. 12 shows an illustrative embodiment of a screen that the processor 62 displays to prompt the operator to enter the identification number of the medium 63. At S260, the type of visit for the test subject is selected from a list of visit types. For example, FIG. 13 shows an illustrative embodiment of a screen that the processor 62 displays to prompt the operator to select one of the visit types.

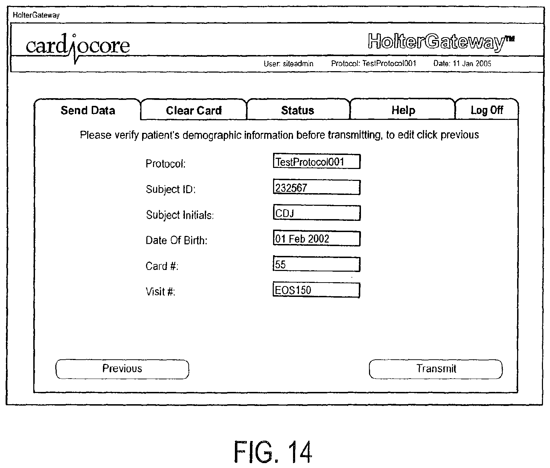

Also, after all of the relevant demographic and other data have been entered, the processor 62 may prompt the operator to verify the data before transmitting it to the central processor 61, as shown in the example in Fig. 14. If the data is correct, the operator selects the "Transmit" option. Otherwise, the operator can select the "Previous" option to page back through the previous screens to correct any data.

If the operator selects the "Transmit" option, then, at S270, the system confirms that the demographic data, the identification number of the portable storage medium 63, the type of visit, etc. are correctly formatted. If an error is detected, then, at S280, an error message is displayed and control returns to S220.

If the demographic data, the identification number of the portable storage medium 63, and the type of visit are correct, all this information is transmitted, along with the electrocardiograph data uploaded from the portable storage medium 63 associated with the test subject, from the remote processor 62 to the central processor 61 via the network 64.

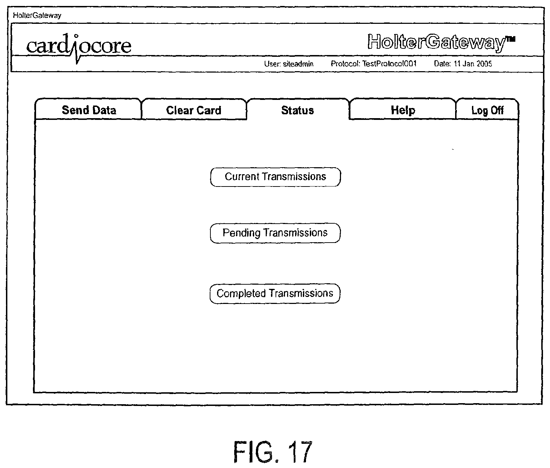

An operator may evaluate the status of various transmissions from the remote processor 62 to the central computer 61. For instance, if the operator selects a "Status" tab, the remote processor 62 may display a screen prompting the operator to select a "Current Transmissions" option, a "Pending Transmissions" option, or a "Completed Transmissions" option as shown in the non-limiting example in Fig. 17.

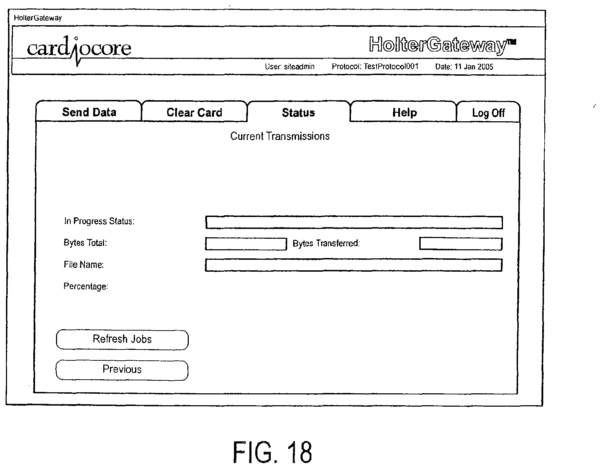

If the operator selects the "Current Transmissions" option, the processor 62 may display a current transmissions screen, as shown in the illustrative example of Fig. 18. In one implementation, the screen displays all of the transmissions from the operator's remote processor 62. As shown in the figure, the screen shows the status of transmission, the total number of bytes to be transferred, the total number of bytes already transferred, the file name being transferred, and the percentage of the file that has been transferred.

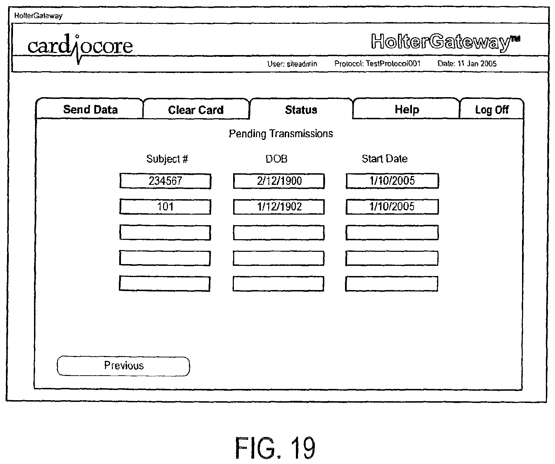

If the operator selects the "Pending Transmissions" option, the processor 62 may display a pending transmissions screen, as shown in the illustrative example of Fig. 19. In one implementation, the screen displays all of the transmissions for a predetermined or specified group of processors 62 or operators. For example, the group may correspond to a plurality of processors 62 in a single drug testing site or a group of processors 63 within a certain geographic area.

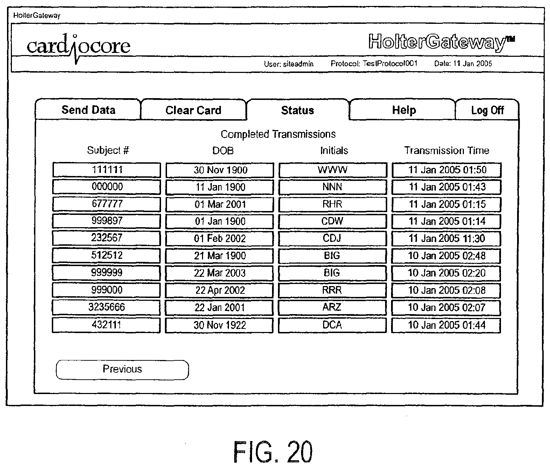

If the operator selects the "Completed Transmissions" option, the processor 62 may display a completed transmissions screen, as shown in the illustrative example of Fig. 20. In one implementation, the screen displays the last ten completed transmissions for the predetermined or specified group.

An alternative, and in some aspects, a more detailed and illustrative, non-limiting embodiment of the invention will be described below. In this embodiment, features of a HolterGateway application will be described. The embodiment entails examples of the design and configuration of hardware, network, operating system, data storage, and software systems that will be employed in the implementation of the application.

The software design is presented from two perspectives. The first formalizes the logical technical divisions that exist between the presentation, application, database, and web services layers of the system. The second defines particular software components according to the functional requirements they address. The design of these software components comprises pieces that reside in one or more of logical layers.

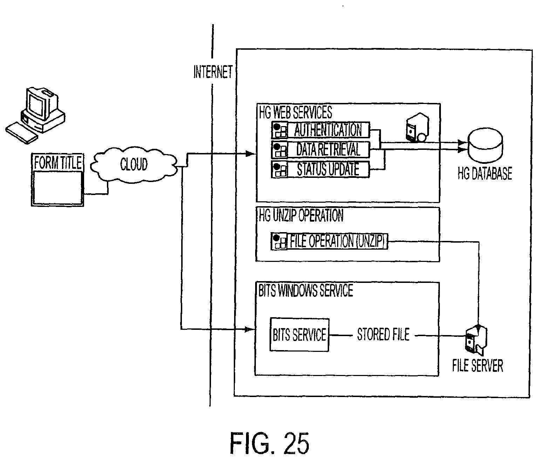

The application can be divided into several services layers, some of which may or may not execute on different computers or processors. In one example, a service layer is a group of software services provided to the application, and in the present embodiment, the HolterGateway application is divided up into four services layers: a Windows Client Application ("HG Client Apps"), a Windows Service application for transmission ("HG Service"), a Web service layer to communicate with database and central ("HG Web Service"), and a Web Administrative application ("HG Web Admin"). FIG. 25 shows a non-limiting example of the system overview.

The HG Client app is executed on the site computer, which may correspond to the remote processor 62 shown in Fig. 6. This application will be the main interface with the user at the site. At a high level, the application will allow the user to log on to the application and initiate a compression and transfer for a portable storage medium 63 (*e.g*., a flash card). After filling in some demographic information, information in the flash card may be zipped into a file and queued for transmission. Also, the application will enable the user to erase a flash card, assuming the flash card "has been released," and to view the status of transmission and HG Service status.

The HG Service will execute on the site computer. This service will be responsible for retrieving compressed files from the queue and sending them to the server. The server may correspond to the central processor 61 shown in Fig. 6. Once the file has been successfully transmitted, the service will unzip in a predefined location and update the database of process information. The demographic information may be embedded in the filename of the compressed file in the following format: USERNAME_GROUPID_CARD#_PROTOCOL_TIMEPOINT_SUBJECTID _SUBJECTINITIALS_DATEBIRTH_CURRENTDATE_GENDER_SERIALNUMBER .zip.

The HG Web service layer may execute on the server (or central processor 61). This layer will be responsible for communicating with the database to authenticate a user, log access for further audit log, and retrieve demographic information corresponding to a protocol list, a timepoint list, a patient list, and a visit list. Also, the layer can unzip a file after successful transmission and update the database, check if the flash card can be released, check if card has been previously transmitted, receive notification when uploads have started and completed, and provide data on the last ten completed transmissions and all pending transmissions. Also, the Web service will be responsible for communicating with the file system and database layer.

The HG Unzip service layer will execute on the server. This layer will be responsible for unzipping a file upon receipt and unzipping the file to a given directory.

The administrative interface will be used to manage the back-end of the HG application. The functions provided will be to maintain the Group/Site (*i.e*., maintain the list of groups or site registered, maintain the protocol per group, maintain the user per group (including the username, password, email, full name, and contact phone number), maintain the visit timepoint per group (including listing the visit number associated with a given group/site), and manage the flash card release. With respect to managing the flash card release, the application may list the card(s) per site/group that have been successfully sent to the server (or central processor 61) and allow them to be released for erasing. Also, the functions of the application enable viewing historical transmissions, listing the transmission for each flash card in a given group, and viewing audit logs.

The HolterGateway may also have functional divisions. Functional divisions are categories of business functions. They describe the implementation of application functionality across technical divisions. Each category is divided up into individual business use cases. Two of the functional divisions in the HolterGateway application include the client application and the administrative interface.

The client application (or HG client app) allows a user at a remote site (or processor 61) to tag and transfer data from a flash card to the server (or central processor 62) and erase data in a card. The functions include transmitting data, erasing card data, and viewing the status of transmissions.

In one implementation of the transmit data function, the user logs on to the HG client app. The application will check the credentials of the user against the list of authorized users and an audit log entry will be performed based on the success or failure of the attempt to log on. Once logged on, the user will be presented with a menu of protocols in a "Select Protocol" screen that is displayed on the remote processor 62. FIG. 21 is an exemplary illustration of the "Select Protocol" screen. If there is only one protocol, the "Select Protocol" screen will be bypassed, and the user will be presented with either a "Clear Card" screen or a "Verify Fax and Card" screen. Fig. 9 shows an example of the "Clear Card" screen, and the "Verify Fax and Card" screen will be described below.

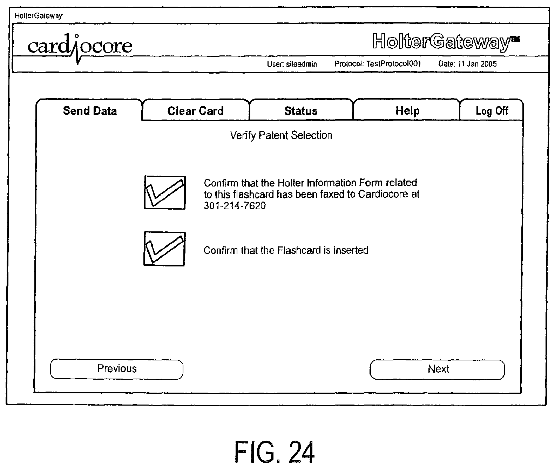

After a protocol is selected (by default or user selection), the user will be presented with the "Clear Card" screen, which contains a list of flash cards that have been processed and are ready to be erased or "cleared." If there are no cards ready to be cleared, the user is presented with the "Verify Fax and Card" screen to verify that a fax has been sent to the location of the server, central processor 61, or central site and that a card has been inserted in the flash card reader of the remote processor 62. Fig. 24 shows an illustrative, non-limiting embodiment of the "Verify Fax and Card" screen.

At the "Clear Card" screen, the user can select a card to be "cleared" by selecting the button with that card's number and clicking the "Clear Card" button. In order for the card's files to be deleted, the volume number of the card currently inserted in the reader must match the volume number stored by the system for that card number. Also, as shown in Fig. 9, the "Clear Card" screen may be displayed by clicking on the "Clear Card" tab.

At the "Verify Fax and Card" screen, the user is required to check that the form has been faxed to a central location (*e.g*., CardioCore) and check that a flash card is inserted in the reader. If the user clicks the "Next" option shown in Fig. 24 and a flash card is not detected in the reader, an error message is displayed and the user is not allowed to continue.

Then, the user specifies whether the flash card contains information regarding a new or existing patient. For example, the remote processor 62 may display the screen shown in Fig. 22 prompting the user to select the "New Patient" option or the "Existing Patient" option. If the user selects the "New Patient" option, the user must enter data for the Subject ID, Subject Initials, Date of Birth, Flash Card Number, Gender, and Visit Number. In one implementation, the processor 62 may display the screens similar to those shown in Figs. 10-13 and 23 to enter such information.

After all of the demographic and other data is entered and if the user clicks on the "Send Data" tab shown in Fig. 13, the processor 62 may display a "Transmit" screen prompting the user to verify the input information. Fig. 14 shows a non-limiting example of such a screen. If the information is correct, the user can click the "Transmit" option to request transmission of data. If the data has been previously transmitted or is currently being transmitted, the user will receive an appropriate message and the data will not be transmitted. If the data on the card has not been previously transmitted, the flash card data will be compressed and placed in a designated "queue" folder for the Windows service to process and physically transmit the data using BITS (Background Intelligent Transfer Service). Also, the user can select the "Previous" option (Fig. 14) to change any of the input information.

If the user selects the "Existing Patient" option in the screen shown in Fig. 22, the processor 62 may display a "Select Patient" screen prompting the user to choose an existing patient from a list and to verify that the selected patient is the patient associated with the data on the flash card. Fig. 15 shows an example of the "Select Patient" screen, and Fig. 16 shows an example of a screen prompting the user to verify the selected patient. Then, the user enters a flash card number, selects a visit and transmits the data as described above.

When a user, who has a certain level of authorization (such as a system administrator), clicks on the "Status Tab," the processor 62 displays a "Status" screen. If a user, who does not have the certain level of authorization, clicks on the tab, the processor will display the "Current Transmissions" screen described below.

Fig. 17 shows a non-limiting example of the "Status" screen. As shown in the figure, the "Status" screen has a "Current Transmissions" option, a "Pending Transmissions" option, and a "Completed Transmissions" option.

If the user selects the "Current Transmissions" option, the "Current Transmissions" screen is displayed. Fig. 18 shows an example of the "Current Transmissions" screen. The screen shows ongoing BITS jobs for the user's computer only. Clicking on one of the BITS jobs shows progress of that transfer job.

If the user selects the "Pending Transmissions" option, a "Pending Transmissions" screen is displayed. Fig. 19 shows an example of the "Pending Transmissions" screen. The "Pending Transmissions" screen displays all transmissions that have started but have not completed for the Group ID specified in the configuration file.

If the user selects the "Completed Transmissions" option, a "Completed Transmissions" screen is displayed. Fig. 20 shows an example of the "Completed Transmissions" screen. The "Completed Transmissions" screen displays the last ten completed transmissions for the Group ID specified in the configuration file.

When the user clicks the "Clear Card" tab, the processor 62 displays a "Clear Card" screen, such as the screen shown in Fig. 9. When this screen is displayed, the user can "clear" or delete data on flash cards as described above.

When the user clicks the "Help" tab, a "Help" screen is displayed. The processor 62 may use an Internet browser to display a web page specified in the configuration file as the "Help" screen.

When the user clicks the "Logoff' tab, he or she is prompted to verify that they really want to log off. If the user verifies that he or she would like to log off, the application returns to a "Logon" screen.

The HG Service application is installed on the computer (or remote processor 62) at the remote site. The application is packaged as a "Window Service" with Start/Stop functionality. The advantage of a windows service is to provide the ability to run in the background and guarantees that the process will work even if no user is logged on to the processor 62. The windows service should provide the ability to start/stop processing. When starting, the service will first determine if there is any pending failure by issuing a web service call to the server (or central processor 61). If any pending jobs exist, it will process those first.

Also, the service should scan a pre-defined directory for files queued for transmission to the central processor 61. It will recover any pending jobs and process failure first. Furthermore, it will issue a web service call to tell the server that a transmission is starting to allow recovery and will take the oldest file first in the directory and send it to the server using BITS. In addition, when the transmission is completed, it will issue a web service call that will unzip the files on the server and mark the file as processed successfully and will move the file to a sub-directory called "Complete."

The service should also recover failures by checking the server for uncompleted jobs and will process jobs as described above. In addition, the service will expose the interface to a query progress and show the status. This will be used from the Windows Client App to display the basic status of the application, such as stopped and started.

The web service interface (or HG web service) layer is responsible for communicating with the file system and database. The following functions will be provided: (1) ValidateLogin (for validating a username/password), (2) LogAction (for logging a user action (action login, transmission, card erase)), (3) CheckPreTransmission (for checking if a card has already been transmitted), (4) CheckRelease (for checking if a card can be released), (5) MarkStartUpload (for logging the start of a transmission and returning a transmission ID), (6) MarkEndUpload (for marking an end of transmission and using a transmission ID), (7) Listpendingdownload (for listing the pending downloads that have failed or are not completed (this function may be used by the windows service to recover failed transmission)), (8) ListCompletedDownload (for listing the completed downloads), (9) GetProtocols (for returning the list of protocols for the group, and (10) GetVisits (for returning the list of visit for the group).

Applications on both the client (or processor 62) and server (or processor 61) should be configurable for easy deployment. The following configuration is an example of a parametrizable configuration on the client and server.

With respect to the HG client,
- GroupID specifies the group.
- CFCardPath specifies where the Windows Client will look for patient data.
- WatchPath specifies where the Windows Client should place compressed data for the Windows Service to detect.
- ServiceName specifies the name of the Windows Service so that the Windows Client can activate the service if it is not started.
- EmailFromUser, EmailFromPwd, EmailFromPwd, EmailFromDisplayName, AdminEmail, AdminEmailDisplayName, SMTPServer, and MailerType designate settings for sending email in case of system errors.
- FontName and FontSize determine the base font and size settings for dynamic drawing of image text.
- SubjectIDFieldLength sets the number of textboxes (1-12) to display on the "Subject ID" screen.
- RetrievalWebServiceURL and TransactionalWebServiceURL specify the location of the web services the Windows Client calls to receive or send metadata. WebServiceUser, WebServicePwd, and WebServiceDomain are used to validate against the Web Service server.
- BITSUploadURL sets the Internet location where the compressed raw data patient files are transmitted. BITSUser and BITSPwd are used to validate against the transfer server.
- MyTraceSwitch sets the level of verbosity written to the Event Log in values of zero to four. A value of zero means that only errors will be written to the Event Log; a value of four means that information about the state of Windows Client will be written after almost every user action.

With respect to the HG server:
- GroupID specifies the group.
- WatchPath specifies where the Windows Service should look for compressed data to transmit.
- EmailFromUser, EmailFromPwd, EmailFromPwd, EmailFromDisplayName, AdminEmail, AdminEmailDisplayName, SMTPServer, and MailerType designate settings for sending email in case of system errors.
- RetrievalWebServiceURL and TransactionalWebServiceURL specify the location of the web services the Windows Client calls to receive or send metadata. WebServiceUser, WebServicePwd, and WebServiceDomain are used to validate against the Web Service server.
- BITSUploadURL sets the Internet location where the compressed raw data patient files are transmitted. BITSUser and BITSPwd are used to validate against the transfer server.
- RecoveryInterval sets the period of time that the Windows Service should search for data to transmit in milliseconds. A setting of 30000 means that the Windows Service will check every thirty seconds for new data or for transmissions that were interrupted or failed and need to be recovered.
- MyTraceSwitch sets the level of verbosity written to the Event Log in values of zero to four. A value of zero means that only errors will be written to the Event Log; a value of four means that information about the state of Windows Client will be written after almost every Windows Service action.

With respect to the HG web service, the Connection String identifies the connection string to the database, the FilePath represents the path to uncompressed files, and the Downloaded Files represents the path to where files are downloaded. With respect to the HG Unzip Service, the PathToWatch is the path to look for files, and the PathToUnzip is the path to uncompressed files.

A non-limiting example of hardware architecture for the embodiment will be described below. The production architecture may comprise the client (or remote site or processor 62). The client may include a Windows XP PC with a hard drive having 10 Gb of memory, a 512Mb RAM, and BITS 1.5 or greater installed or Windows XP SP2. The server (or central processor 61) may include a Windows Server 2003 with a hard drive having 30 Gb of free space, BITS 2.0 installed, and the Web Service layer installed on the web server.

The server portion of HolterGateway may be installed on Lyra. Also, the following may be performed. BITS may be installed on LYRA, and three new virtual directories may be created:
**HGBITS** is used by Windows Client to transfer information. This virtual directory is enabled to use BITS. Also HGBITS can be set to accept Basic Authentication and force SSL for encryption.
**HGWS** is used to host the web services program. The web services may be made of two different methods: Retrieval.asmx and Transactional.asmx. The web service may be secured using Basic Authentication and requiring SSL Encryption.
**HGADMIN** is used for the administrative interface. The application may be secured using form based authentication.

Most of the configuration is stored in configuration file. In one implementation, only a few options need to be changed.

With respect to the windows client configuration, the file HolterGatewayWindowsClient.exe.config may be used to maintain the configuration of the Windows Apps. Also, CFPath= E:\\ may be the path to the Flash Card, and the GroupID=1 value may need to be changed for each Group or site.

With respect to the windows service configuration, the file HolterGatewayTransferService.exe.config may be used to maintain the configuration of the Windows Apps. Also, the GroupID=1 value may need to be changed for each Group or site.

With respect to the web service configuration, the service may have a web.config file which contains the configuration used by the web service to communicate with the SQL database and to know where to retrieve the files. In one embodiment, these options should not be changed.

```
 ConnectionString="packet size=4096;data source=lyra;initial
 catalog=HoltergatewayProd; Integrated Security=SSPI"
 UploadPath = E:\HGBITS.
```

With respect to the unzip service configuration, the web service may have an UnzipService.exe.config file which contains the configuration used by the unzip to read and unzip the files.

```
 PathToWatch, E:\\HGBITS
 PathToUnzip, E:\\HGDATA
```

With respect to the admin interface configuration, the interface may have a web.config file which constrains the configuration used by the admin interface to communicate with the SQL database.

```
 ConnectionString="packet size=4096;data source=vela;initial
 catalog=HoltergatewayProd; Integrated Security=SSPI"
```

The foregoing description of the exemplary embodiments of the invention has been presented for purposes of illustration and description. It is not intended to be exhaustive or to limit the invention to the precise form disclosed, and modifications and variations are possible in light of the above teachings or may be acquired from practice of the invention. The exemplary embodiments were chosen and described in order to explain the principles of the invention and its practical application to enable one skilled in the art to utilize the invention in various exemplary embodiments and with various modifications as are suited to the particular use contemplated.

Thus, while only certain exemplary embodiments of the invention have been specifically described herein, it will be apparent that numerous modifications may be made thereto without departing from the spirit and scope of the invention. Further, acronyms are used merely to enhance the readability of the specification and claims. It should be noted that these acronyms are not intended to lessen the generality of the terms used and they should not be construed to restrict the scope of the claims to the exemplary embodiments described therein.

## Claims

1. A method for transfer of electrocardiograph data between a remote processor (62) connected to a central processor (61) via a network (64) wherein identification information concerning a test subject associated with the electrocardiograph data is entered, the method being **characterized by**:
recording demographic data associated with the test subject on a portable storage medium (63) containing the electrocardiograph data;
coupling (S100) the portable storage medium (63) containing the electrocardiograph data to the remote processor (62);
extracting the electrocardiograph data from the portable storage medium (63) and transmitting (S300) the identification information and the electrocardiograph data from the remote processor (62) to the central processor (61); and
transmitting (S400) a release message from the central processor (61) to the remote processor (62) to enable erasure of the electrocardiograph data from the portable storage medium (63), wherein the transmission of the release message is subsequent to the successful storage of the electrocardiograph data on the central processor (61).

2. The method as claimed in claim 1, wherein the entering of identification information comprises selecting a drug test protocol associated with the test subject (S210).

3. The method as claimed in claim 1, wherein the entering of identification information further comprises at least one of entering an identification number of the portable storage medium (S250) and entering a visit type (S260).

4. The method as claimed in claim 3, wherein the visit type is at least one of a baseline visit, a first day visit, a second day visit and a screening visit.

5. The method as claimed in claim 1, wherein the method further comprises, subsequent to the entry of identification information, checking to determine if the entered identification information follows a predefined format (S280).

6. The method as claimed in claim 1, wherein the identification information comprises at least.one of a test subject's identification number, date of birth, initials and gender.

7. The method as claimed in claim 1, wherein the entering of identification information comprises selecting a test subject from a list of test subjects stored on the remote processor (S240).

8. The method as claimed in claim 1, wherein the method further comprises monitoring transmissions of identification information and electrocardiograph data from the remote processor to the central processor.

9. An apparatus for transferring electrocardiograph data from a portable storage medium, the apparatus comprising:
a portable storage medium for storing electrocardiograph data and demographic data associated with a test subject;
a remote processor (62) for extracting the electrocardiograph data from the portable storage medium (63) and transmitting identification information concerning the test subject associated with the electrocardiograph data and the extracted electrocardiograph data;
a central processor (61) for storing the extracted electrocardiograph data and transmitting a release message to the remote processor (62) to enable erasure of the electrocardiograph data from the portable storage medium (63); and
a network (64) connecting the remote processor (62) and the central processor (61),
wherein the central processor (61) is configured to receive the identification information and electrocardiograph data transmitted by the remote processor (62) via the network, and the remote processor (62) is configured to receive the release message from the central processor (61) via the network.

10. The apparatus as claimed in claim 9, wherein the identification information comprises a drug test protocol associated with the test subject.

11. The apparatus as claimed in claim 10, wherein the identification information further comprises at least one of demographic data associated with the test subject for storage on the remote processor, an identification number of the portable storage medium (63) and a visit type.

12. The apparatus as claimed in claim 10, wherein the visit type is at least one of a baseline visit, a first day visit, a second day visit and a screening visit.

13. The apparatus as claimed in claim 10, wherein the identification information comprises at least one of a test subject's identification number, date of birth, initials and gender.

14. The apparatus as claimed in claim 9, wherein the remote processor (62) monitors transmissions of identification information and electrocardiograph data to the central processor (61).

## Patentansprüche

1. Verfahren zur Übertragung von Elektrokardiographie-Daten zwischen einem Remote-Prozessor (62), welcher mit einem Zentralprozessor (61) über ein Netzwerk (64) verbunden ist, wobei Identifikationsinformation betreffend eine Testperson, die mit den Elektrokardiographie-Daten in Zusammenhang steht, eingegeben wird, wobei das Verfahren **gekennzeichnet ist durch**:
Aufzeichnen demographischer Daten, die mit der Testperson in Zusammenhang stehen, auf einem tragbaren Speichermedium (63), welches die Elektrokardiographie-Daten enthält;
Verbinden (S100) des tragbaren Speichermediums (63), welches die Elektrokardiographie-Daten enthält, mit dem Remote-Prozessor (62);
Extrahieren der Elektrokardiographie-Daten aus dem tragbaren Speichermedium (63) und Übermitteln (S300) der Identifikationsinformation und der Elektrokardiographie-Daten von dem Remote-Prozessor (62) an den Zentralprozessor (61); und
Übermitteln (S400) einer Release-Nachricht von dem Zentralprozessor (61) an den Remote-Prozessor (62), um ein Löschen der Elektrokardiographie-Daten von dem tragbaren Speichermedium (63) zu ermöglichen, wobei die Übermittlung der Release-Nachricht auf die erfolgreiche Speicherung der Elektrokardiographie-Daten auf dem Zentralprozessor (61) folgt.

2. Verfahren gemäß Anspruch 1, wobei die Eingabe von Identifikationsinformation die Auswahl eines Medikament-Testprotokolls, das mit der Testperson in Zusammenhang steht, umfasst (S210).

3. Verfahren gemäß Anspruch 1, wobei die Eingabe von Identifikationsinformation außerdem die Eingabe einer Identifikationsnummer des tragbaren Speichermediums (S250) und/oder die Eingabe eines Visiten-Typs (S260) umfasst.

4. Verfahren gemäß Anspruch 3, wobei der Visiten-Typ eine Baseline-Visite, eine Tag-1-Visite, eine Tag-2-Visite und/oder eine Screening-Visite ist.

5. Verfahren gemäß Anspruch 1, wobei das Verfahren, nach der Eingabe der Identifikationsinformation, außerdem eine Überprüfung umfasst, um zu bestimmen, ob die eingegebene Identifikationsinformation einem vorgegebenen Format folgt (S280).

6. Verfahren gemäß Anspruch 1, wobei die Identifikationsinformation die Identifikationsnummer, das Geburtsdatum, die Initialen und/oder das Geschlecht einer Testperson umfasst.

7. Verfahren gemäß Anspruch 1, wobei die Eingabe der Identifikationsinformation das Auswählen einer Testperson aus einer Liste von Testpersonen, die auf dem Remote-Prozessor gespeichert ist, umfasst (S240).

8. Verfahren gemäß Anspruch 1, wobei das Verfahren außerdem die Überwachung der Übermittlungen von Identifikationsinformation und Elektrokardiographie-Daten von dem Remote-Prozessor an den Zentralprozessor umfasst.

9. Vorrichtung zur Übertragung von Elektrokardiographie-Daten von einem tragbaren Speichermedium, wobei die Vorrichtung umfasst:
ein tragbares Speichermedium zum Speichern von Elektrokardiographie-Daten und demographischen Daten, die mit einer Testperson in Zusammenhang stehen;
einen Remote-Prozessor (62) zum Extrahieren der Elektrokardiographie-Daten aus dem tragbaren Speichermedium (63) und Übermitteln von Identifikationsinformation betreffend die Testperson, die mit den Elektrokardiographie-Daten in Zusammenhang steht, und der extrahierten Elektrokardiographie-Daten;
einen Zentralprozessor (61) zum Speichern der extrahierten Elektrokardiographie-Daten und Übermitteln einer Release-Nachricht an den Remote-Prozessor (62), um ein Löschen der Elektrokardiographie-Daten von dem tragbaren Speichermedium (63) zu ermöglichen; und
ein Netzwerk (64), welches den Remote-Prozessor (62) und den Zentralprozessor (61) miteinander verbindet,
wobei der Zentralprozessor (61) so konfiguriert ist, dass er die Identifikationsinformation und die Elektrokardiographie-Daten, die durch den Remote-Prozessor (62) über das Netzwerk übermittelt werden, empfängt, und der Remote-Prozessor (62) so konfiguriert ist, dass er die Release-Nachricht von dem Zentralprozessor (61) über das Netzwerk empfängt.

10. Vorrichtung gemäß Anspruch 9, wobei die Identifikationsinformation ein Medikament-Testprotokoll, das mit der Testperson in Zusammenhang steht, umfasst.

11. Vorrichtung gemäß Anspruch 10, wobei die Identifikationsinformation außerdem demographische Daten, die mit der Testperson in Zusammenhang stehen, zur Speicherung auf dem Remote-Prozessor, eine Identifikationsnummer des tragbaren Speichermediums (63) und/oder einen Visiten-Typ umfasst.

12. Vorrichtung gemäß Anspruch 10, wobei der Visiten-Typ eine Baseline-Visite, eine Tag-1-Visite, eine Tag-2-Visite und/oder eine Screening-Visite ist.

13. Vorrichtung gemäß Anspruch 10, wobei die Identifikationsinformation die Identifikationsnummer, das Geburtsdatum, die Initialen und/oder das Geschlecht einer Testperson umfasst.

14. Vorrichtung gemäß Anspruch 9, wobei der Remote-Prozessor (62) die Übermittlung der Identifikationsinformation und der Elektrokardiographie-Daten an den Zentralprozessor (61) überwacht.

## Revendications

1. Un procédé de transfert de données électrocardiographiques entre un processeur distant (62) raccordé à un processeur central (61) par l'intermédiaire d'un réseau (64), dans lequel des informations d'identification concernant un sujet soumis à test associées aux données électrocardiographiques sont entrées, le procédé étant **caractérisé par** :
l'enregistrement de données démographiques associées au sujet soumis à test sur un support à mémoire portatif (63) contenant les données électrocardiographiques,
le couplage (S100) du support à mémoire portatif (63) contenant les données électrocardiographiques au processeur distant (62),
l'extraction des données électrocardiographiques du support à mémoire portatif (63) et la transmission (S300) des informations d'identification et des données électrocardiographiques du processeur distant (62) vers le processeur central (61), et
la transmission (S400) d'un message de libération du processeur central (61) vers le processeur distant (62) de façon à permettre l'effacement des données électrocardiographiques du support à mémoire portatif (63), la transmission du message de libération étant subséquente à la conservation en mémoire réussie des données électrocardiographiques sur le processeur central (61).

2. Le procédé selon la revendication 1, dans lequel l'entrée d'informations d'identification comprend la sélection d'un protocole de test de médicaments associé au sujet soumis à test (S210).

3. Le procédé selon la revendication 1, dans lequel l'entrée d'informations d'identification comprend en outre au moins une opération parmi l'entrée d'un numéro d'identification du support à mémoire portatif (S250) et l'entrée d'un type de visite (S260).

4. Le procédé selon la revendication 3, dans lequel le type de visite est au moins une visite parmi une visite préliminaire, une visite de premier jour, une visite de deuxième jour et une visite de dépistage.

5. Le procédé selon la revendication 1, dans lequel le procédé comprend en outre, suite à l'entrée d'informations d'identification, une vérification visant à déterminer si les informations d'identification entrées respectent un format prédéfini (S280).

6. Le procédé selon la revendication 1, dans lequel les informations d'identification comprennent au moins un élément parmi un numéro d'identification, une date de naissance, des initiales et un genre du sujet soumis à test.

7. Le procédé selon la revendication 1, dans lequel l'entrée d'informations d'identification comprend la sélection d'un sujet soumis à test à partir d'une liste de sujets soumis à test conservée en mémoire sur le processeur distant (S240).

8. Le procédé selon la revendication 1, dans lequel le procédé comprend en outre la surveillance de transmissions d'informations d'identification et de données électrocardiographiques du processeur distant vers le processeur central.

9. Un appareil de transfert de données électrocardiographiques à partir d'un support à mémoire portatif, l'appareil comprenant :
un support à mémoire portatif destiné à la conservation en mémoire de données électrocardiographiques et de données démographiques associées à un sujet soumis à test,
un processeur distant (62) destiné à l'extraction des données électrocardiographiques du support à mémoire portatif (63) et à la transmission d'informations d'identification concernant le sujet soumis à test associées aux données électrocardiographiques et des données électrocardiographiques extraites,
un processeur central (61) destiné à la conservation en mémoire des données électrocardiographiques extraites et à la transmission d'un message de libération au processeur distant (62) de façon permettre l'effacement des données électrocardiographiques du support à mémoire portatif (63), et
un réseau (64) raccordant le processeur distant (62) et le processeur central (61),
le processeur central (61) étant configuré de façon à recevoir les informations d'identification et les données électrocardiographiques transmises par le processeur distant (62) par l'intermédiaire du réseau, et le processeur distant (62) étant configuré de façon à recevoir le message de libération à partir du processeur central (61) par l'intermédiaire du réseau.

10. L'appareil selon la revendication 9, dans lequel les informations d'identification comprennent un protocole de test de médicaments associé au sujet soumis à test.

11. L'appareil selon la revendication 10, dans lequel les informations d'identification comprennent en outre au moins un élément parmi des données démographiques associées au sujet soumis à test destinées à une conservation en mémoire sur le processeur distant, un numéro d'identification du support à mémoire portatif (63) et un type de visite.

12. L'appareil selon la revendication 10, dans lequel le type de visite est au moins une visite parmi une visite préliminaire, une visite de premier jour, une visite de deuxième jour et une visite de dépistage.

13. L'appareil selon la revendication 10, dans lequel les informations d'identification comprennent au moins un élément parmi un numéro d'identification, une date de naissance, des initiales et un genre du sujet soumis à test.

14. L'appareil selon la revendication 9, dans lequel le processeur distant (62) surveille des transmissions d'informations d'identification et de données électrocardiographiques vers le processeur central (61).
